(19) Europäisches Patentamt
European Patent Office
Office européen des brevets

(11) Veröffentlichungsnummer: **0 620 212 A1**

(12) # EUROPÄISCHE PATENTANMELDUNG

(21) Anmeldenummer: **94102106.5**

(22) Anmeldetag: **11.02.94**

(51) Int. Cl.5: **C07C 271/34**, C07C 271/36, C08G 18/10, C08G 18/32, C07D 251/34

(30) Priorität: **10.04.93 DE 4311922**

(43) Veröffentlichungstag der Anmeldung: **19.10.94 Patentblatt 94/42**

(84) Benannte Vertragsstaaten: **CH DE FR GB IT LI NL**

(71) Anmelder: **HÜLS AKTIENGESELLSCHAFT**

**D-45764 Marl (DE)**

(72) Erfinder: **Lomölder, Rainer, Dr.**
**Gigasstrasse 40 b**
**D-48153 Münster (DE)**
Erfinder: **Paulen, Wilfried, Dr.**
**Keplerstrasse 13**
**D-45657 Recklinghausen (DE)**
Erfinder: **Schmitt, Felix, Dr.**
**Tiroler Weg 2**
**D-45701 Herten (DE)**
Erfinder: **Wolf, Elmar, Dr.**
**Stauffenbergstrasse 7**
**D-45661 Recklinghausen (DE)**

(54) **Polyamine, Verfahren zu ihrer Herstellung sowie ihre Verwendung als Epoxidharzhärter.**

(57) Die Erfindung betrifft neue Polyamine folgender Zusammensetzung:

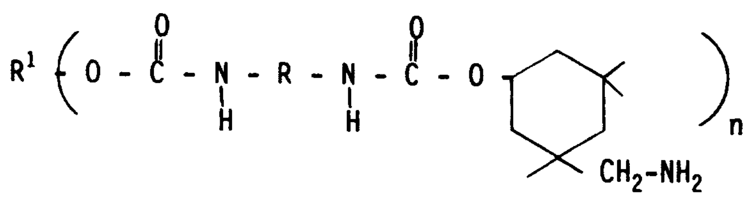

(I)

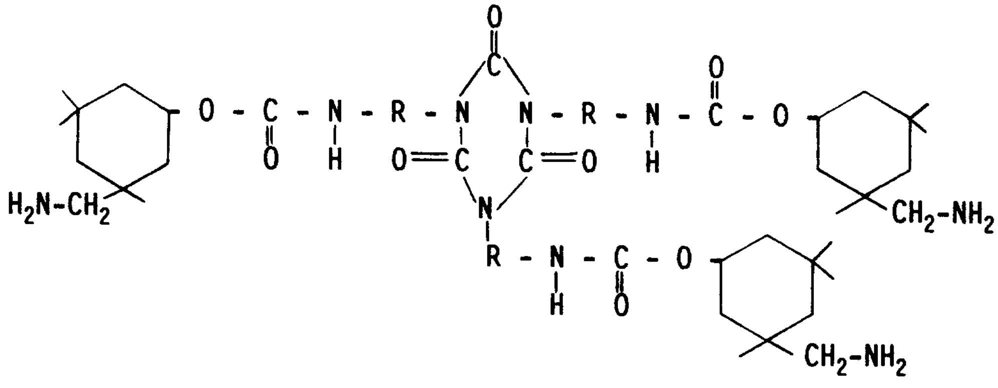

(II),

wobei 1 ≦ n ≦ 6 ist,

R: den KW-Rest eines (cyclo)aliphatischen oder aromatischen Diisocyanates mit 6 - 20 C-Atomen oder deren Trimerisate,

$R^1$: wenn 2 ≦ n ≦ 6, einen n-wertigen organischen Rest, wie er durch Entfernung von n OH-Gruppen aus

EP 0 620 212 A1

einer gegebenenfalls Ethersauerstoffatome enthaltenden Polyhydroxylverbindung mit einem durchschnittlichen Molgewicht zwischen 60 und 5 000 entsteht,
wenn n : 1

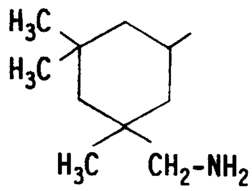

darstellen.

Sie betrifft ferner ein Verfahren zur Herstellung der Polyamine, wobei man bestimmte Diisocyanate oder Addukte von Diisocyanaten mit Polyolen oder deren Trimersate mit der Schiffschen Base folgender Zusammensetzung:

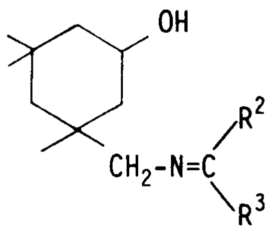
(I - Sch B) ,

wobei
$R^2$ = H, $C_1$-$C_8$-Alkyl
$R^3$ = $C_1$-$C_8$-(cyclo)-Alkyl sind,
umsetzt und nach erfolgter Reaktion die gebildeten Polyazomethine (Poly-Schiffsche Basen) mit überschüssigem Wasser vollständig hydrolysiert und die gebildete Carbonylverbindung abdestilliert.

Die erfindungsgemäßen Polyamine werden zur Härtung von Epoxidharzen eingesetzt.

Die vorliegende Erfindung betrifft neue Polyamine, ein Verfahren zu ihrer Herstellung sowie ihre Verwendung als Epoxidharzhärter.

Polyamine, speziell Diamine, haben einmal als Ausgangskomponenten zur Herstellung von Polyamiden eine herausragende Bedeutung erlangt, zum anderen werden sie in großem Umfang zur Härtung von Epoxidharzen, speziell auf Bisphenol A-Basis, eingesetzt.

Während niedermolekulare Polyamine technisch leicht zugänglich sind und daher in großer Auswahl zur Verfügung stehen, ist das Problem der Herstellung von höhermolekularen Di- und Polyaminen bisher noch nicht befriedigend gelöst.

Aufgabe der vorliegenden Erfindung war es deshalb, ein einfaches, reproduzierbares Verfahren zur Herstellung von höhermolekularen Di- und Polyaminen zur Verfügung zu stellen.

Ein seit langem bekanntes Verfahren zur Herstellung solch maßgeschneiderter Polyamine besteht darin, daß NCO-Präpolymere mit Aminoalkoholen, deren $NH_2$-Gruppe gegenüber der NCO-Gruppe in Form einer Aldimin- oder Ketimingruppe geschützt ist, umgesetzt und anschließend hydrolysiert werden. Bei diesem Verfahren ist es wichtig, daß die Aminogruppe von der OH-Gruppe durch mindestens 6 C-Atome getrennt ist, da bei einem geringeren Abstand der beiden Gruppen, vor allem dann, wenn sich 5-, 6-, und 7-Ringe bilden können, sich diese auch bevorzugt bilden und die gewünschten Schiffschen Basen nur als Nebenprodukt anfallen. Es war deshalb bis jetzt nicht möglich, Aminoalkohole, deren funktionelle Gruppen 2 - 4 C-Atome von einander entfernt sind, zur Herstellung von Polyaminen einzusetzen.

Überraschenderweise wurde parallel gefunden, daß bestimmte Aminoalkohole, deren funktionelle Gruppen 4 C-Atome von einander getrennt sind, mit Aldehyden und Ketonen nicht wie erwartet einen heterocyclischen 7-Ring ergeben, sondern Azomethine (Schiffsche Basen), wenn es sich beim Aminoalkohol um folgende Verbindung handelt:

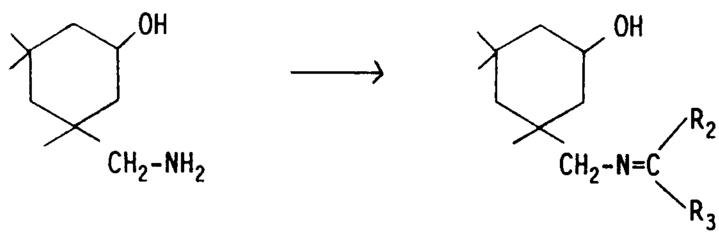

Sch B

Diese Schiffschen Basen sind Gegenstand einer gesonderten Anmeldung P .. .. ... gleichen Zeitranges.

In Lösung der Aufgabe konnten durch Umsetzung vorstehender Schiffschen Basen und Diisocyanatpräpolymeren (Addukte von Diisocyanaten mit Polyolen oder Trimerisate von Diisocyanaten) und anschließender Hydrolyse die gewünschten Di- und Polyamine in einfacher Weise hergestellt werden.

Gegenstand der Erfindung sind daher Polyamine der folgenden Zusammensetzung:

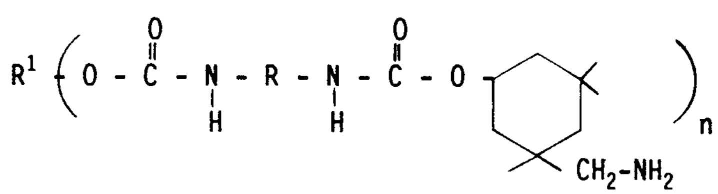

(I)

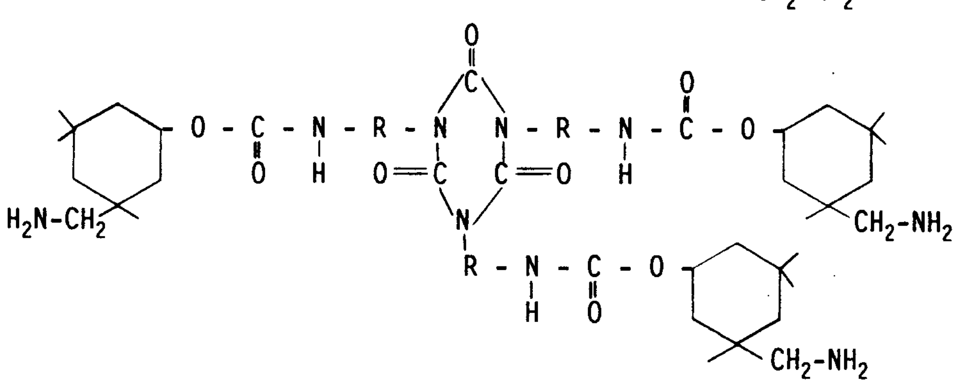

(II),

wobei $1 \leqq n \leqq 6$ ist,

R: den KW-Rest eines (cyclo)aliphatischen oder aromatischen Diisocyanates mit 6 - 20 C-Atomen oder deren Trimerisate,

$R^1$: wenn $2 \leqq n \leqq 6$, einen n-wertigen organischen Rest, wie er durch Entfernung von n OH-Gruppen aus einer gegebenenfalls Ethersauerstoffatome enthaltenden Polyhydroxylverbindung mit einem durchschnittlichen Molgewicht zwischen 60 und 5 000 entsteht,
wenn n : 1

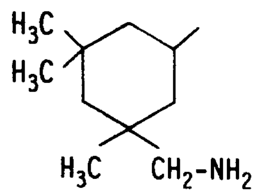

darstellen.

Die erfindungsgemäßen Verbindungen zeichnen sich durch ein Molgewicht von 500 -6 000 und einen basischen Amingehalt von 0,3 - 4 mmol $NH_2$/g aus. Ihre Konsistenz ist in einem weiten Bereich variierbar. Während die Ethergruppen enthaltenden erfindungsgemäßen Polyamine eine Viskosität von 50 000 - $10^6$ mPa•s aufweisen, handelt es sich bei den Isocyanuratgruppen aufweisenden Polyaminen um hochschmelzende Verbindungen mit einem Schmelzpunkt > 150 °C.

Ein weiterer Gegenstand der vorliegenden Erfindung ist ein Verfahren zur Herstellung der Polyamine nach Anspruch 1, welches dadurch gekennzeichnet ist, daß Diisocyanate oder Addukte von Diisocyanaten mit Polyolen (Ia) oder deren Trimerisate (IIa)

OCN - R - NCO

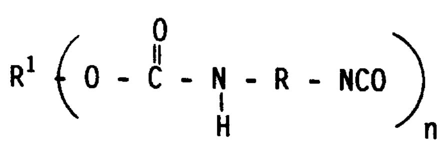

**(I a)**

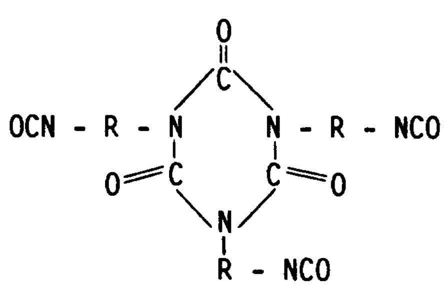

**(II a)** ,

wobei n, R und $R_1$ die in Anspruch 1 angegebene Bedeutung haben, mit der Schiffschen Base folgender Zusammensetzung:

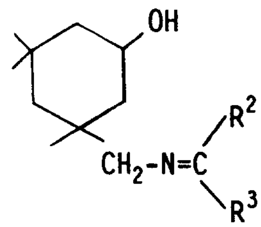

(I - Sch B) ,

wobei
$R^2$ = H, $C_1$-$C_8$-Alkyl
$R^3$ = $C_1$-$C_8$-(cyclo)-Alkyl
im NCO : OH-Äquivalenzverhältnis von 1 : 1 umgesetzt werden und nach erfolgter Reaktion die gebildeten Polyazomethine (Poly-Schiffsche Basen) der Formel I b und II b

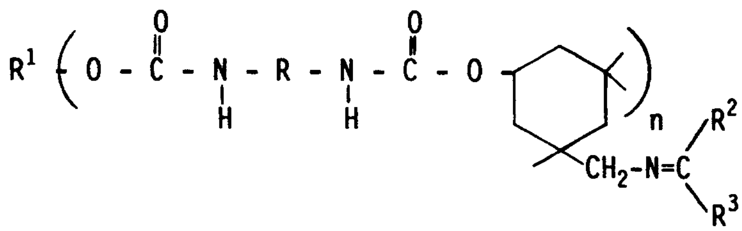

(I b)

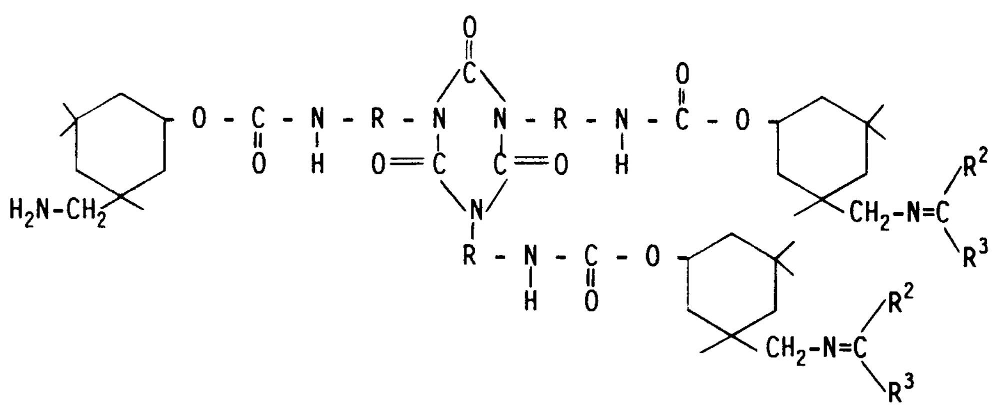

(II b)

mit überschüssigem Wasser vollständig hydrolysiert und die gebildete Carbonylverbindung abdestilliert wird.

Die Umsetzung erfolgt je nach Aggregatzustand des eingesetzten NCO-Präpolymeren bzw. Diisocyanats in Substanz oder in aprotischen Lösungsmitteln bei Temperaturen von 20 - 80 °C. Bei niedrig viskosen NCO-Präpolymeren kann auf Lösungsmittel verzichtet werden; bei hochviskosen und festen NCO-Präpolymeren muß dagegen in Lösung gearbeitet werden. Als Lösungsmittel kommen im Prinzip alle in Frage, die keine gegenüber NCO-Gruppen reaktiven funktionellen Gruppen enthalten. Besonders geeignet haben sich Ketone, wie z. B. Aceton, Methylethylketon und aromatische Kohlenwasserstoffe, wie z. B. Toluol, erwiesen.

Die für das erfindungsgemäße Verfahren eingesetzten Isocyanatpräpolymeren (Ia, II a) werden nach an sich bekannten Methoden durch Umsetzung von Polyhydroxylverbindungen mit Diisocyanaten bzw. durch Trimerisierung von Diisocyanaten hergestellt.

Als Polyhydroxylverbindungen kommen Polyole mit einem Molgewicht von 60 - 600, wie z. B. Ethylenglykol, Hexandiol, Di- und Triethylenglykol, Neopentylglykol, Trimethylolpropan, Octandecandiol, $C_{36}$-Diol in Frage. Bevorzugt eignen sich Polyetherpolyole mit einem Molgewicht von 200 - 5 000, mit 2 - 5, vorzugsweise 2 - 3 Hydroxylgruppen. Die erfindungsgemäß in Frage kommenden, Hydroxylgruppen aufweisenden Polyether sind solche der an sich bekannten Art und werden z. B. durch Polymerisation von

Epoxiden, wie Ethylenoxid, Propylenoxid, Tetrahydrofuran, Styroloxid mit sich selbst, z. B. in Gegenwart von Lewis-Säuren, wie z. B. $BF_3$ oder durch Anlagerung dieser Epoxide, gegebenenfalls im Gemisch oder nacheinander, an Starterkomponenten mit reaktionsfähigen Wasserstoffatomen, wie Wasser, Alkohole und Amine hergestellt. Weiterhin werden OH-Gruppen enthaltende Polybutadiene für die Isocyanatpräpolymeren eingesetzt.

Als Ausgangskomponente zur Herstellung der für das erfindungsgemäße Verfahren benötigten Isocyanatpräpolymeren (I a) kommen (cyclo)aliphatische, araliphatishe, aromatische Diisocyanate in Betracht, wie sie z. B. von W. Siefken in Justus Liebigs Annalen der Chemie, 562, S. 75 - 136, beschrieben werden, beispielsweise 1.6-Hexamethylendiisocyanat, 2-Methylpentamethylendiisocyanat, 1.12-Dodecandiisocyanat, Isophorondiisocyanat, Tetramethylxylendiisocyanat, 2.4- und 2.6-Toluylendiisocyanat und Diphenylmethan-2.4'- und/oder -4.4'-diisocyanat.

Die Isocyanatpräpolymeren (I a) werden in der Regel so hergestellt, daß auf 1 OH-Äquivalent des Polyols 2 NCO-Äquivalente des Diisocyanats in bekannter Weise miteinander umgesetzt werden. Die so hergestellten Isocyanatpräpolymeren enthalten je nach Molgewicht noch ca. 2 - 8 % freies Diisocyanat. In manchen Fällen hat es sich als zweckmäßig erwiesen, Isocyanatpräpolymere mit einem Monomergehalt < 0,5 % für das erfindungsgemäße Verfahren einzusetzen. Solche monomerarmen Isocyanatpräpolymeren werden so hergestellt, daß in einer 1. Stufe das Diisocyanat in großem Überschuß mit dem Polyol zur Reaktion gebracht, und in einer 2. Stufe das nicht umgesetzte Diisocyanat durch Dünnschichtdestillation vom Reaktionsprodukt abgetrennt wird. Die so hergestellten Isocyanatpräpolymeren enthalten unabhängig von ihrem Molgewicht < 0,5 % Diisocyanat.

Die für das erfindungsgemäße Verfahren eingesetzten Isocyanatpräpolymeren II a werden in bekannter Weise durch Trimerisierung der bereits für die Herstellung der Isocyanatpräpolymeren I a aufgezählten Diisocyanate, wie z. B. in DE-PS 29 16 201 beschrieben, hergestellt.

Als Reaktionskomponente für die Isocyanatpräpolymeren (I a, II a) wird nach dem erfindungsgemäßen Verfahren folgender, aminblockierter Alkohol eingesetzt:

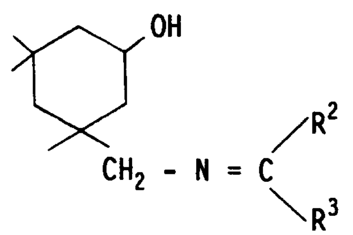

(I Sch B)

wobei zur Blockierung der $NH_2$-Funktion prinzipiell alle Aldehyde bzw. Ketone in Frage kommen. Als besonders geeignet haben sich von den Aldehyden: Acetaldehyd, Propionaldehyd, n-Butyraldehyd, i-Butyraldehyd, von den Ketonen: Mehtylethylketon, Methyl-n-propylketon, Diethylketon, Methyl-isobutylketon, Diisobutylketon erwiesen.

Nach erfolgter Umsetzung des Isocyanatpräpolymeren (I a, II a) mit dem aminblockierten Alkohol (I-Sch B) wird nun in einem 2. Verfahrensschritt das Reaktionsgemisch der 1. Stufe hydrolysiert:

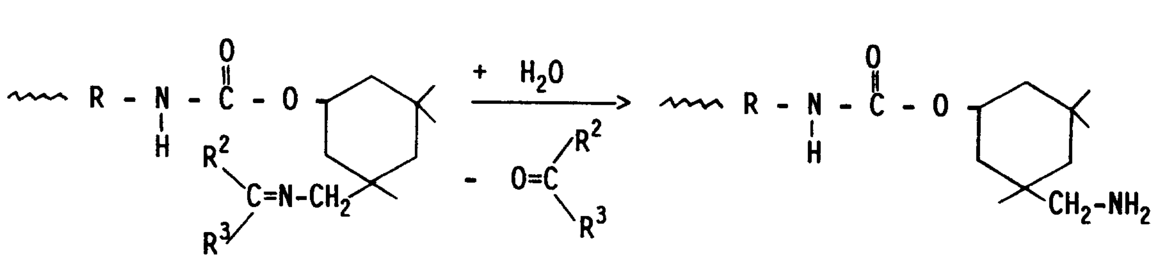


Die Abspaltung der Carbonylverbindungen wird so durchgeführt, daß das blockierte Polyamin, gegebenenfalls in Gegenwart von 0,1 - 0,5 % Emulgatoren, mit überschüssigem $H_2O$ (2- 3fache Menge des blockierten Polyamins) unter intensiver Rührung erhitzt wird, wobei gleichzeitig $H_2O$, die freigesetzte Carbonylverbindung und gegebenenfalls das Lösungsmittel bei Normaldruck abdestilliert werden.

Zur Entfernung der letzten Reste $H_2O$ wird das Polyamin im Vakuum bei 100 - 140 °C ca. 2 - 4 h weiter erhitzt. Die so hergestellten Polyamine enthalten keine Azomethin-Gruppen mehr; es liegen aus-

schließlich primhäre $NH_2$-Gruppen vor. Der $H_2O$-Gehalt liegt bei 0,1 - 0,6 %.

Ein weiterer Gegenstand der vorliegenden Erfindung ist die Verwendung der erfindungsgemäßen Verbindungen zur Härtung von Epoxidharzen.

Als Epoxidharze kommen im Prinzip alle in Frage, die $\geqq$ 2 Epoxidgruppen pro Molekül enthalten; als besonders geeignet haben sich die Epoxidharze auf Bisphenol A- und F-Basis erwiesen. Da es sich bei den erfindungsgemäßen Polyaminen um hochviskose bzw. feste Substanzen handelt, kommen sie in der Regel lösungsmittelhaltig zum Einsatz. Als Lösungsmittel kommen Benzylalkohol, Nonylphenol, Toluol, Xylol, N-Methylpyrrolidon in Frage. Die erfindungsgemäßen Polyamine können entweder allein oder in Kombination mit einem Diamin oder primären Monoamin mit dem Epoxidharz, gegebenenfalls in Gegenwart der bekannten Katalysatoren, wie 2,4,6-Trisdimethylaminomethylphenol, Salicylsäure oder Dimethylbenzylamin, zur Reaktion gebracht werden. Durch geeignete Wahl der Kombination der Komponenten: Polyamin, Diamin und Monoamin lassen sich (bei konstant gelassenem EP-Harz) bei Raumtemperatur beliebig flexible Beschichtungen mit guten mechanischen Eigenschaften herstellen.

Bei den mit den erfindungsgemäßen Polyaminen einsetzbaren Diaminen kommen z. B. folgende in Frage: Ethylendiamin, 1.2-(1.3)-Diaminopropan, 1.3-(1.4)-Diaminobutan, 3-(Isopropyl-amino)-propylamin, 1-Cyclohexylamino-3-aminopropan, 1.4-Diaminocyclohexan, 1.3-Diaminocyclohexan, Isophorondiamin, 2-Methylpentamethylendiamin,2.2.4(2.4.4)-Trimethylhexamethylendiamin, Hexamethylendiamin, N-Aminoethylpiperazin, m-Xylylendiamin. Bei den Monoaminen, die mit den erfindungsgemäßen Polyaminen und gegebenenfalls mit den genannten Diaminen zur Herstellung der erfindungsgemäßen Beschichtungen zum Einsatz kommen, handelt es sich in der Regel um folgende: Decylamin, Dodecylamin, Tridecylamin, Butoxypropylamin, Hexoxypropylamin, 3-(2-Ethylhexoxy)-proylamin, Lauryloxypropylamin, Diethylaminopropylamin.

**Experimenteller Teil**

A. Herstellung der Schiffschen Base

Ein Gemisch aus 342 Gew.-T. 3-Aminomethyl-3,5,5-trimethyl-cyclohexanol und 260 Gew. -T. Methylisobutylketon wird am Wasserabscheider unter Rückfluß so lange gekocht, bis sich 36 Gew.-T. Wasser abgeschieden haben. Das überschüssige MIBK wird dann unter vermindertem Druck abdestilliert. In der Regel kann auf eine weitere Reinigung verzichtet werden. Die OH-Zahl des Reaktionsproduktes beträgt 221 mg KOH/g.

B. Herstellung der erfindungsgemäßen Polyamine

1. 254 Gew.-T. der Schiffschen Base A und 560 Gew.-T. eines NCO-Präpolymeren, das nach bekannten Verfahren aus 444 Gew.-T. IPDI und 650 Gew.-T. eines Polytetrahydrofurandiols mit durchschnittlichem Molgewicht 650 hergestellt wurde, werden bei 50 °C so lange zusammen erhitzt, bis kein NCO mehr nachgewiesen werden kann (ca. 20 h). Zu dieser Poly-Schiffschen Base werden 2 000 Gew. -T. $H_2O$ gegeben und unter kräftigem Rühren zum Sieden erhitzt, wobei das $H_2O$ kontinuierlich bei Normaldruck abdestilliert wird. Nach Beendigung der Reaktion bei Normaldruck wird zur Vervollständigung der Umsetzung noch ca. 4 h bei 100 °C und 0,1 Torr weiter erhitzt.

Das so erhaltene Reaktionsprodukt enthält ausschließlich primäre Aminogruppen. Durch NMR-Messung kann keine Azomethingruppe mehr nachgewiesen werden. Der basische Amingehalt beträgt 1,3 mmol/g; die Viskosität beträgt bei 25 °C $1{,}2 \cdot 10^6$ mPa•s. Der $H_2O$-Gehalt beträgt 0,3 %.

2. 254 Gew. -T. der Schiffschen Base A und 737 Gew. -T. eines NCO-Präpolymeren, das nach bekannten Verfahren aus 444 Gew.T. IPDI und 1 000 Gew.-T. eines Polytetrahydrofurandiols mit durchschnittlichem Molgewicht von 1 000 hergestellt wurde, werden analog Beispiel 1 umgesetzt und anschließend mit 2 500 Gew.-T. $H_2O$ unter den in Beispiel 1 angegebenen Reaktionsbedingungen hydrolysiert.

Das Reaktionsprodukt hat einen Gehalt an basischem Amin von 1.05 mmol $NH_2$/g und eine Viskosität von $10^6$ mPa•s (bei 25 °C). Der $H_2O$-Gehalt beträgt 0,5 %.

3. 254 Gew.-T. der Schiffschen Base A und 1 313 Gew.-T. eines Isocyanatpräpolymeren, das nach bekannten Verfahren aus 444 Gew.-T. IPDI und 2 000 Gew.-T. eines bifunktionellen Polypropylenglykols mit einem durchschnittlichen Molgewicht von 2000 hergestellt wurde, werden analog Beispiel 1 umgesetzt und anschließend mit 3 000 Gew.-T. $H_2O$ unter den im Beispiel 1 angegebenen Reaktionsbedingungen hydrolysiert.

Das Reaktionsprodukt hat einen Gehalt an basischem Amin von 0,6 mmol $NH_2$/g und eine Viskosität von $1{,}6 \cdot 10^5$ mPa•s (bei 25 °C). Der $H_2O$-Gehalt beträgt 0,4 %.

4. 244 Gew.-T. trimeres IPDI mit 17,2 % NCO (VESTANAT T 1890, Verkaufsprodukt der Hüls AG) werden in 300 Gew.-T. Aceton gelöst und mit 254 Gew.-T. der Schiffschen Base A analog Beispiel 1 umgesetzt und anschließend mit 1 500 Gew.-T. $H_2O$ unter den im Beispiel 1 angegebenen Reaktionsbedingungen hydrolysiert.

Das Reaktionsprodukt hat einen Gehalt an basischem Amin von 1,4 mmol $NH_2$/g und einen Schmelzbereich von 180 - 190 °C; Der $H_2O$-Gehalt beträgt 0,2 %.

C. Herstellung von flexiblen Epoxidharzbeschichtungen

I. Zusammensetzung der Härter

Härter 1: 80 Gew.-T. des Polyamins B. 3,
20 Gew.-T. Benzylalkohol und
15,7 Gew.-T. IPD
werden gemischt
NH-aktiv Äquivalentgewicht: 250
Viskosität (b. 23 °C) mPa•s: 5990

Härter 2: 80 Gew.-T. des Polyamins B. 3,
20 Gew.-T. Benzylalkohol und
45,9 Gew.-T. 2-Ethylhexoxypropylamin
werden gemischt
NH-aktiv Äquivalentgewicht: 250
Viskosität (b. 23 °C) mPa•s: 437

Härter 3: 70 Gew.-T. des Polyamins B. 1,
30 Gew.-T. Benzylalkohol und
11,4 Gew.-T. IPD
werden gemischt
NH-aktiv Äquivalentgewicht: 250
Viskosität (b. 23 °C) mPa•s: $10{,}8.10^3$

Härter 4: 70 Gew.-T. des Polyamins B. 1,
30 Gew.-T. Benzylalkohol
33,6 Gew.-T. 2-Ethylhexoxypropylamin
werden gemischt
NH-aktiv Äquivalentgewicht: 250
Viskosität (b. 23 °C) mPa•s: $1{,}33.10^3$

C. II Härtung von EPIKOTE® 828 mit den erfindungsgemäßen Härtern ( $\nabla_{O}$ : NH = 1 : 1); 7 Tage, Raumtemperatur

| Härter Zs.-setzung Gew.-T. | | Zugfestigkeit $Nmm^{-2}$ (DIN 53 504) | Zug-Dehnung % (DIN 53 504) | Reißfestigkeit $Nmm^{-2}$ (DIN 53 504) | Reiß-Dehnung % (DIN 53 504) | Weiterreißwiderstand $Nmm^{-1}$ (DIN 53 515) | Shore A | Shore D |
|---|---|---|---|---|---|---|---|---|
| Härter 1 | Härter 2 | | | | | | | |
| 80 | 20 | 19,4 ± 0,8 | 118,7 ± 17,7 | 19,4 ± 0,8 | 118,9 ± 17,3 | 50,4 ± 3,9 | | 60 |
| 50 | 50 | 15,7 ± 0,7 | 174,7 ± 11,3 | 15,7 ± 0,7 | 174,7 ± 11,3 | 53,7 ± 2,5 | | 55 |
| 25 | 75 | 11,6 ± 0,4 | 276,8 ± 8,9 | 11,6 ± 0,4 | 276,8 ± 8,9 | 33,0 ± 1,9 | | 39 |
| 20 | 80 | 10,1 ± 0,2 | 277,4 ± 6,2 | 10,1 ± 0,2 | 277,5 ± 6,1 | 36,0 ± 1,2 | | 37 |
| Härter 3 | Härter 4 | | | | | | | |
| 100 | - | 25,7 ± 0,7 | 15,6 ± 1,4 | 21,8 ± 0,3 | 38 ± 2,5 | 73,7 ± 9,2 | - | 70 |
| 75 | 25 | 17,5 ± 0,9 | 113,3 ± 9,2 | 17,5 ± 0,9 | 113,3 ± 9,2 | 50,2 ± 1,8 | - | 63 |
| 50 | 50 | 14,6 ± 1,1 | 212,1 ± 1,1 | 14,6 ± 1,1 | 212,1 ± 12,5 | 45,8 ± 3,9 | - | 45 |
| 25 | 75 | 14,6 ± 1,1 | 327,5 ± 12,5 | 9,3 ± 0,5 | 327,5 ± 12,5 | 30,7 ± 1,8 | 85 | 35 |
| - | 100 | 1,0 ± 0 | 185,9 ± 7,8 | 1,0 ± 0 | 186.3 ± 7,6 | 16,2 ± 2,8 | 61 | - |

## Patentansprüche

**1.** Neue Polyamine folgender Zusammensetzung:

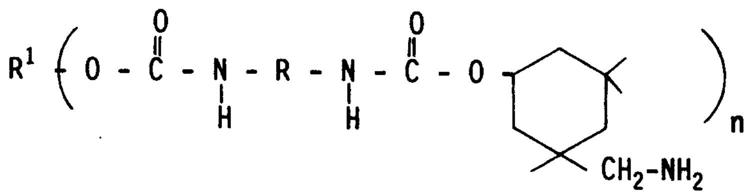

**(I)**

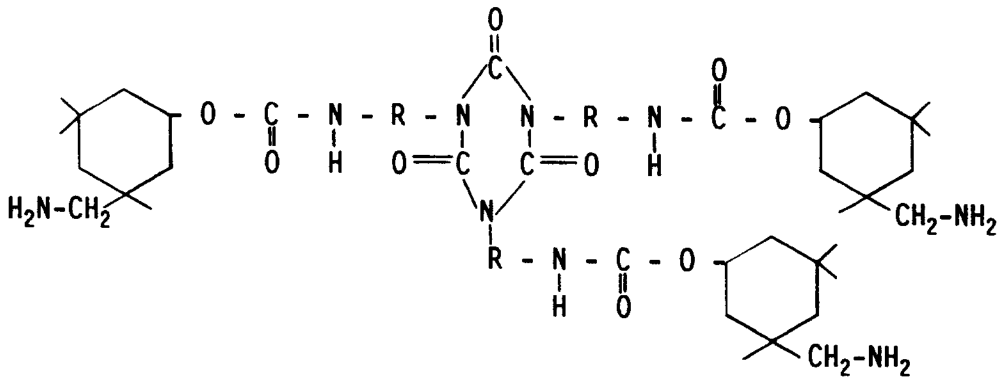

**(II)**,

wobei 1 ≦ n ≦ 6 ist,

R: den KW-Rest eines (cyclo)aliphatischen oder aromatischen Diisocyanates mit 6 - 20 C-Atomen oder deren Trimerisate,

$R^1$: wenn 2 ≦ n ≦ 6, einen n-wertigen organischen Rest, wie er durch Entfernung von n OH-Gruppen aus einer gegebenenfalls Ethersauerstoffatome enthaltenden Polyhydroxylverbindung mit einem durchschnittlichen Molgewicht zwischen 60 und 5 000 entsteht,
wenn n : 1

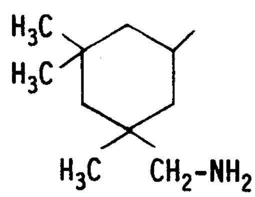

darstellen.

**2.** Verfahren zur Herstellung der Polyamine nach Anspruch 1,
dadurch gekennzeichnet,
daß Diisocyanate oder Addukte von Diisocyanaten mit Polyolen (Ia) oder deren Trimerisate (II a):

$OCN - R - NCO$

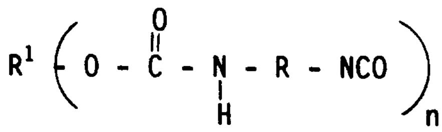

(I a)

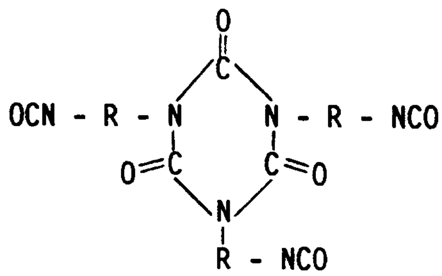

(II a),

wobei n, R und $R_1$ die in Anspruch 1 angegebene Bedeutung haben, mit der Schiffschen Base folgender Zusammensetzung:

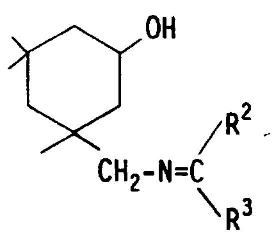

(I - Sch B) ,

wobei

$R^2$ = H, $C_1$-$C_8$-Alkyl

$R^3$ = $C_1$-$C_8$-(cyclo)-Alkyl sind,

im NCO : OH-Äquivalenzverhältnis von 1 : 1 umgesetzt werden,und nach erfolgter Reaktion die gebildeten Polyazomethine (Poly-Schiffsche Basen) der Formel I b und II b

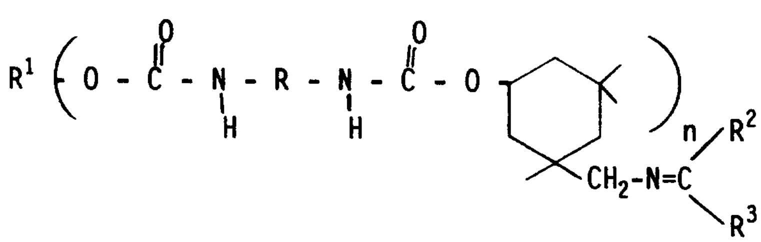

(I b)

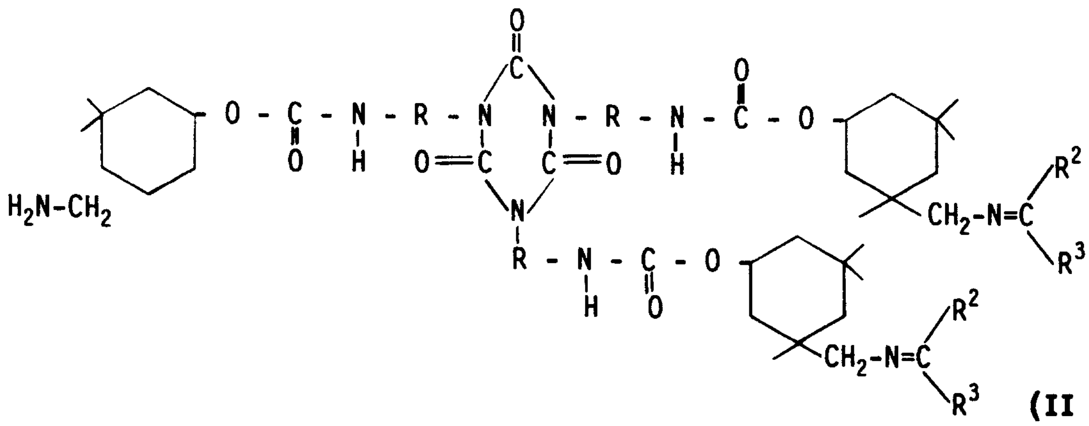

(II b)

mit überschüssigem Wasser vollständig hydrolysiert, und die gebildete Carbonylverbindung abdestilliert wird.

3. Verfahren zur Herstellung der Polyamine nach Anspruch 2,
dadurch gekennzeichnet,
daß die Umsetzung gegebenenfalls in Lösemitteln erfolgt.

4. Verwendung der Polyamine gemäß Anspruch 1 zur Härtung von Epoxidharzen.

Europäisches Patentamt

# EUROPÄISCHER RECHERCHENBERICHT

Nummer der Anmeldung
EP 94 10 2106

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| Y | FR-A-2 328 006 (BAYER AG)<br>* Seite 2, Zeile 4 - Seite 3, Zeile 15 *<br>* Seite 5, Zeile 30 - Zeile 32; Ansprüche 1,2 * | 1-4 | C07C271/34<br>C07C271/36<br>C08G18/10<br>C08G18/32<br>C07D251/34 |
| Y | US-A-3 352 913 (K. SCHMITT)<br>* Anspruch 2 * | 1-4 | |
| A | FR-A-2 362 171 (BAYER AG)<br>* Seite 14, Zeile 20 - Zeile 25; Ansprüche * | 1-4 | |
| | | | RECHERCHIERTE SACHGEBIETE (Int.Cl.5) |
| | | | C07C<br>C08G<br>C07D |

Der vorliegende Recherchenbericht wurde für alle Patentansprüche erstellt

| Recherchenort | Abschlußdatum der Recherche | Prüfer |
|---|---|---|
| DEN HAAG | 28. Juni 1994 | Seufert, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)